# EUROPEAN PATENT SPECIFICATION

(11) **EP 1 689 249 B1**
(45) Date of publication and mention of the grant of the patent: **23.07.2008**
(21) Application number: 04813134.6
(22) Date of filing: 02.12.2004
(51) Int. Cl.: A23K 1/18, A23K 1/16, A23L 1/29, A61K 31/195, A61P 1/14, A61P 3/04

(54) **DIETARY NON-ESSENTIAL AMINO ACID TYROSINE REGULATES THE BODY WEIGHT OF ANIMALS THROUGH REGULATING THE ANIMAL APPETITE OR FOOD INTAKE**
DIE ORALE AUFNAHME DER NICHT ESSENTIELLEN AMINOSÄURE TYROSIN ALS REGULATIV DES KÖRPERGEWICHTS VON TIEREN DURCH REGULIERUNG DES APPETITES DER TIERE ODER DEREN FUTTERAUFNAHME
TYROSINE D'ACIDE AMINE ALIMENTAIRE NON ESSENTIELLE REGULANT LE POIDS CORPOREL D'ANIMAUX PAR REGULATION DE L'APPETIT OU DE L'INGESTION ALIMENTAIRE DE CEUX-CI

(30) Priority: 02.12.2003 US 725856
(43) Date of publication of application: 16.08.2006
(73) Proprietor: Hill's Pet Nutrition Inc., Topeka, KS 66603 (US)
(72) Inventor: YU, Shiguang, Topeka, KS 66617 (US); MILLER, Cheryl, Lawrence, KS 66047 (US); JEWELL, Dennis, Lawrence, KS 66047 (US); NADEAU, Doug, Topeka, KS 66617 (US)
(74) Representative: Daniels, Jeffrey Nicholas
(86) International application number: PCT/US2004/040770
(87) International publication number: WO 2005/055738

(56) References cited:
- WO-A-01/11990
- CH-A- 554 644
- US-A- 5 817 695
- US-A1- 2002 094 969
- US-A1- 2003 138 547
- HARVIE MN, CAMPBELL IT, HOWELL A, THATCHER N: "Acceptability and tolerance of a low tyrosine and phenylalanine diet in patients with advanced cancer - a pilot study" JOURNAL OF HUMAN NUTRITION AND DIETETICS, vol. 15, 2002, pages 193-202, XP002318304
- MORRIS JG, ROGERS QR: "Assessment of the nutritional adequacy of pet foods through the life cycle" JOURNAL OF NUTRITION, vol. 124, 1994, pages 2520S-2534S, XP009044193 USA
- LACY MP, VAN KREY HP, DENBOW DM, SIEGEL PB, CHERRY JA: "Amino acid regulation of food intake in domestic fowl" NUTRITION AND BEHAVIOR, vol. 1, 1982, pages 65-74, XP009044184
- AVRAHAM Y, HAO S, MENDELSON S, BERRY EM: "Tyrosine improves appetite, cognition, and exercise tolerance in activity anorexia" MED. SCI. SPORTS EXERC., vol. 33, no. 12, 2001, pages 2104-2110, XP009044140

## Description

It is reported that over half of the human adult population in the United States are overweight or obese (Pi-Sunyer, F. X. NHLBI Obesity Education Initiative Expert Panel on the identification, evaluation, and treatment of overweight and obesity in adults - The evidence report. Obese. Res., 1998; 6:51S-209S; Must A., Spadano, J., Coakley, E.H., et al., The disease burden associated with overweight and obesity. JAMA 1999; 282:1523-9). The prevalence of obesity in adults in the United States has almost doubled between 1960 and 1994 (Flegal, K., Carrol, M., Kuczmarski, R., Johnson, C. Overweight and obesity in the United States: prevalence and trends, 1960-1994. Int. J. Obes. 1998; 22:39-47).

In a survey of domestic cats presented to veterinarians, 25% of the cats are overweight or obese (Scarlett, J.M., Donoghue, S., Saidla, J., Wills, Overweight cats: prevalence and risk factors, J. hit J Obes Relat Metab Disord. 1994, 8 Suppl 1:S22-8). A similar percentage of obese dogs was reported in the United Kingdom (Edney, A.T.B., Smith, P. M., Study of obesity in dogs visiting veterinary practices in the United Kingdom. Vet Rec. 1986; 118:391-396). Obesity contributes to many chronic diseases, such as hypertension, cardiovascular diseases, and diabetes mellitus, in humans as well as companion animals.

Tyrosine is a non-essential amino acid for domestic cats as it can be produced by cats through phenylalanine hydroxylation. AAFCO Cat Food Nutrient Profiles for Adult Maintenance for phenylalanine is 0.42% dry matter basis (DM) and for phenylalanine and tyrosine is 0.88% (DM) (Official Publication 2003, Association of American Feed Control Officials, Inc.). AAFCO recommendations are based on studies in growing kittens for maximum growth. Recent studies found that the AAFCO recommendation for tyrosine and phenylalanine does not support maximum hair color production in growing kittens (Yu, S., Rogers, QR, Morris, J. G. Effect of low levels of dietary tyrosine on the hair color of cats. J. Small Anim. Prac. 2001, 42:176-180 and Anderson, PJB et. al. Cats require more dietary phenylalanine or tyrosine for melanin deposition in hair than for maximal growth. J. Nutr. 2002, 132:2037-2042). The minimal dietary phenylalanine and tyrosine concentration for hair color deposition was recommended to be 1.8% in growing kittens. It was also reported that hyperactivity and ataxia were noted in kittens fed a diet with a combination of tyrosine and phenylalanine less than 1.6% (Anderson, PJB et, al. Cats require more dietary phenylalanine or tyrosine for melanin deposition in hair than for maximal growth. J. Nutr. 2002, 132:2037-2042). At the present, the minimal requirement of phenylalanine and tyrosine for grown or adult cats has not been experimentally determined. It is known that many nutrient requirements differ between a growing animal and a grown or mature animal.

Tyrosine injection improves appetite, cognition, and exercise tolerance in activity anorexia in mice (Avraham, Y., et al. (2001) Tyrosine Improves Appetite, Cognition, And Exercise Tolerance In Activity Anorexia. Med. Sci. Sports Exerc., 33:2104-2110),
US 2002/0094969 describes a pharmacologic therapy for treatment of obesity which requires selective serotonin reuptake inhibitor medication and may include use of cysteine with tyrosine, 5-hydroxytryptophan, vitamin C, vitamin B6 and calcium.
WO 01/11990 describes a composition for an infant formula having a low threonine content. The protein source for the infant formula may include tyrosine.
US 5817695 describes a nutritional product for cancer patients, comprising high fat, low carbohydrate and an elemental amino acid profile, including L-tyrosine, having a selected amino acid imbalance for suppressing tumour growth.
Harvie et al in J. Hum. Nutr. Dietet., 15, 193-202 (2002) describes a pilot study of the acceptability and tolerance of a low tyrosine and phenylalanine diet in patients with advanced cancer.

### SUMMARY OF THE INVENTION

The present invention provides an edible composition which comprises tyrosine in an amount not to exceed about 0.4% by weight on a dry matter basis, for use in reducing the body weight of an animal.

We found that changing tyrosine concentration in animal foods, preferably pet foods using the inventive formulation process induces body weight loss in animals through reducing food intake or increasing satiety. This invention allows the appropriate concentration of tyrosine without the previously reported negative effects.

An animal as described in this application can be any mammal or bird. The animals can be but are not limited to humans, house pets, farm animals and birds The birds include but are not limited to domestic birds, canaries, parrots, or commercial birds, such as but not limited to chickens, ducks, turkeys, etc. The pets include but are not limited to canine, feline, ferrets, and rodents. The farm animals include but are not limited to horses, hogs, cows, sheep, goats etc. The rodents include but are not limited to hamsters, mice, rats, guinea pigs, gerbils, rabbits, hedge hogs, chinchillas etc.

We have found that reducing the tyrosine content in food will cause a reduction in body weight, while increasing the tyrosine level in food will cause the body weight to remain constant or increase depending upon the amount of the increase of tyrosine.

An object of the invention is to develop a weight management system which comprises administering tyrosine in the desired amount to change and maintain the body weight of an animal. If the animal is overweight, the animal would be fed a low level tyrosine food composition. If the animal is underweight, the animal would be fed a high level tyrosine food composition.

Accordingly, the present invention provides an animal food product useful in controlling daily tyrosine or tyrosine and/or phenylalanine intake to control food intake and energy intake and to control the body weight. It includes, but is not limited to, food, treats, supplements and their combination, which affects voluntary food intake and body weight in animals.

Another object of the invention is an animal supplement, animal snack or animal treat which comprises tyrosine in an amount less than 0.4% by weight DM.

A further obj ect is an edible composition for reducing the body weight of an animal which comprises tyrosine in an amount up to about 0.4 % by weight on a DM.
The invention also provides an edible composition which comprises on a dry matter basis
(a) from about 0.01 % to less than about 0.4% by weight of tyrosine,
(b) from about 0.1 % up to the toxic level of phenylalanine,
(c) from about 7% to about 70% by weight of protein,
(d) from about 1% to about 60% by weight of fat,
(e) from 0 to 90% by weight of carbohydrate,
(f) from 0 to about 40% by weight of dietary fiber, and
(g) from 0 to about 15% by weight of nutritional balancing agents.
The invention also provides use of tyrosine for the production of an animal food for controlling the body weight of an animal, which comprises determining if the animal is an overweight or underweight animal and feeding the overweight animal an animal food which comprises this edible composition, with a low tyrosine content, for a period until said animal reaches the desired weight; or feeding the underweight animal an animal food with a high tyrosine content based on a dry matter basis of at least 1.2% by weight for a period until said animal reaches the desired weight.

### BRIEF DESCRIPTION OF THE FIGURES

Figure 1 illustrates the body weight change of felines according to study 2.

Figure 2 illustrates the food intake change of felines according to study 2.

Figure 3 illustrates the body weight change of felines according to study 3.

Figure 4 illustrates the food intake change of felines according to study 3.

### DETAILED DESCRIPTION OF THE INVENTION

We have demonstrated that an animal's body weight can be controlled and regulated by changing dietary tyrosine concentration, a non-essential amino acid. We believe the mechanism of action to be within reward areas of the central nervous system (CNS), and since these CNS areas are similar among many species, we believe that this technology will also be effective in the canine species and other mammals, In addition, since phenylalanine is an essential amino acid and is oxidized to tyrosine in its metabolic pathway, modification of the dietary phenylalanine level can also lead to the same effect on the body weight as modification of dietary tyrosine concentration.

The foods in this invention have changes in the tyrosine concentration through formulation of ingredients selected for their specific tyrosine concentration. Examples of selected low tyrosine ingredients are gelatin, lungs, whey and grains. To increase tyrosine concentration, selected ingredients such as muscle proteins, casein or tyrosine per so can be used.

Tyrosine is administered as in a food, a treat, a supplement or their combination. To reduce food intake and body weight, tyrosine in a low level is preferably in an amount from about 0.01 to less than about 0,4%by weight and more preferably from about 0.08 to about 0.38 % by weight and most preferably from about 0.2 to about 0.36% by weight on dry matter basis,

The food can be dry or wet. The minimum requirements for a nutritionally adequate food composition are:

| Nutrient | Nutrient Content % (Dry Matter Basis) |
|---|---|
| Carbohydrate (nitrogen free extract) weight, | 0 to about 90%, preferably 10 to 50% by |
| Protein | about 7 to about 70%, preferably 30 to 50% by weight, |
| Fat | about 1 to about 60%, preferably 20 to 50% by weight, |
| Total dietary fiber weight, | 0 to about 40%, preferably 5 to 25% by |
| Nutritional balancing agents such as vitamins and minerals. | 0 to about 15%, 0.1 to 4% by weight |

All the percentages used in this application are by weight on a dry matter basis unless otherwise specified.

The only ingredients required for a food are protein, fat, tyrosine, and phenylalanine. Some animals additionally require carbohydrates and fibers. The preferred compositions will vary according to the animal as discussed below.

The nutritional balancing agents include vitamins and minerals. The nutritional balancing agents should be included in amounts required to avoid deficiency. The National Research Council (NRC) gives the recommendations for farm animals in Nutrient Requirements of Swine: 10th Revised Edition, 1998, Nutrient Requirements of Poultry, Ninth Revised Edition, 1994, Nutrient Requirements of Horses, Fifth Revised Edition, 1989, and so on for other species, National Academy Press, 2101 Constitution Avenue, NW, Washington, DC 20418. AAFCO gives the recommendation for dogs and cats (American Feed Control Officials, Inc., Official publication 2003, page 138-140). Generally the nutritional balancing agents are present in an amount from 0 to about 15% and preferably about 0.1 to about 4%, on a dry matter basis.

A treat or an animal snack is usually defined as a product that is given to an animal that entices them to eat usually during a non-meal time. For example, a bone would be considered a treat for a canine. Nutritional treats have the same requirements as a food. Non-nutritional treats are any compositions that are non-toxic. The only required ingredient for a treat or animal snack is the tyrosine in an amount of at least 0.01% by weight on a dry matter basis.

A supplement as defined in American Feed Control Officials, Inc. Official Publication 2003 at page 220, is a feed used with another to improve the nutritive balance or performance of the total and intended to be:
(1) Fed undiluted as a supplement to other feeds; or,
(2) Offered free choice with other parts of the ration separately available; or
(3) Further diluted and mixed to produce a complete feed. The only required ingredient for a supplement is the tyrosine in an amount of at least 0.01% by weight on a dry matter basis.

For the new food composition containing a low level of tyrosine, the tyrosine is present on a dry matter basis by weight in an amount from about 0.01 % to about 0.4 %, more preferably from about 0.08% to about 0.38% and more preferably from about 0.2% to about 0.36%, based on the content % on a dry matter basis. Phenylalanine is an essential amino acid for cats and other animals. Therefore, a minimal amount of phenylalanine required by the animal at its particular life stage is necessary in the diet. Preferably the phenylalanine is present in an amount from 0.1% by weight up to just below the toxic level of the phenylalanine, and preferably from about 0.2 % to about 10 % by weight, more preferably from about 0.42 to about 3% by weight, and most preferably from about 0.5% to about 0.9 % by weight, based on the content % on a dry matter basis.

For the maintenance composition to maintain energy balance of a cat with optimal body weight, the tyrosine is present on a dry basis in the food from an amount of about 0.4 to about 1.2% by weight and preferably from about 0.45% to about 1.0% by weight. The food composition would be essentially the same as the low tyrosine composition except for the tyrosine level. Added tyrosine can replace any non-essential nutrients such as glycine and fiber. In example 1 glycine is replaced by the additional tyrosine.

For the high tyrosine food composition used for animals underweight, the tyrosine is present on a dry basis in the food from an amount of at least about 1.2% by weight, preferably about 1.2% by weight to just below the toxic level of tyrosine, more preferably from about 1.2% by weight to about 8% and even more preferably from about 1.5% by weight to about 3.5% by weight and most preferably from about 1.9% by weight to about 3% by weight. The food composition would be essentially the same as the low tyrosine composition except for the tyrosine level. Added tyrosine can replace any non-essential nutrients such as glycine and fiber. In example 1 glycine is replaced by the additional tyrosine.

The animal food compositions of the present invention may contain ingredients such as meat, meat by-products, other animal protein sources and grains as the food source. By meat, it means the flesh of cattle, swine, sheep, goat, and other mammals as well as poultry and fish. Meat by-products include, but are not limited to lungs, kidneys, brain, livers, and stomachs and intestines freed of their contents. Additionally, meat, meat by-products, and other animal and / or plant protein source mixtures are suitable for use in the animal food of this invention. The nutrient ingredients may also include amounts of cereal grains such as wheat, corn, barley, rice, and vegetable fiber sources such as cellulose, beet pulp, peanut hulls or soy fiber.

The animal food product according to the invention can contain one of food additives providing functionality beneficial to weight management. Examples include non-fermentable fiber, carnitine, chromium, picolinate and the like.

The animal food product of the present invention may further contain additives commonly known in the art in an amount which does not impair the purpose and effect provided by the invention. Examples of such additives are substances with a stabilizing effect and processing aids.

Substances with a stabilizing effect may be added to increase the shelf life of the product of the invention by supplementing or reinforcing the effect of the physical methods used for increasing the shelf life according to the invention. Examples of substances with a stabilizing effect optionally contained in the animal food product of the invention are preservatives, antioxidants, synergists and sequestrants, packaging gases, stabilizers, emulsifiers, thickeners, gelling agents, and humectants. Examples for the emulsifiers and/or thickening agents are gelatin, cellulose ethers, starch, starch esters, and modified starches.

In preparing the animal food product of the present invention with the low tyrosine content, the nutrient composition is adjusted so that the concentration of tyrosine is present in the animal food product of the present invention at a concentration of about 0.01 % up to less than 0.4%, preferably from about 0.08 to about 0.38% and more preferably from 0.2 to 0.36% based on the content % on a dry matter basis.

The animal food products of the present invention can be prepared in the canned or wet form using conventional pet food processes. One embodiment begins by mixing ground animal and poultry proteinaceous tissues with the remaining ingredients which can include fish oils, cereal grains and other nutritionally balancing ingredients and special purpose additives such as vitamin and mineral mixtures, inorganic salts, cellulose and beet pulp bulking agents and the like. Water sufficient for processing is also added. A vessel suitable for heating while blending the components is used.

Heating of the ingredient mix may be effected in any suitable manner as, for example, by direct steam injection or by using a vessel fitted with a heat exchanger. Following the addition of the last ingredient, the mixture is heated to a temperature typically used in the art. When heated to the appropriate temperature, the heated mixture is then filled into cans. A lid is applied and the container is hermetically sealed. Next, the sealed can is placed into conventional equipment designed to sterilize the contents.

The animal food products of the present invention can be prepared in a dry form using conventional pet food processes. Dry ingredients, including animal and or plant protein sources, grains, and other dry ingredients are ground and mixed together. Moist or liquid ingredients, including fats, oils, and animal protein sources, and water are added to and mixed with the dry mix prior to processing into a kibble or similar dry piece. Most commonly, kibble is formed using the extrusion process where the mixture of dry and wet ingredients is subjected to mechanical work at a high pressure and temperature and forced through small openings and cut off into kibbles by a rotating knife. The kibbles are then dried and may receive one or more topical coatings which may include flavors, fats, oils, powders, and the like.

Kibbles can also be made from the dough using a baking process, rather than extrusion, where the dough is placed into some sort of mold prior to dry heat processing.

Animal treats of the present invention can be prepared by extrusion or baking similar to dry food as is common for commercial pet food. Other processes may also be used to either coat tyrosine on the exterior of existing treat forms, or inject it inside a treat.

The overweight animal is preferably fed the low tyrosine composition for a period until the overweight animal reaches the desired weight. The period is preferably at least 4 weeks, more preferably at least 6 weeks, and most preferably at least 8 weeks. Again, this period depends on the animal's original weight and the desired optimal weight trying to be achieved.

The underweight animal is preferably fed the high tyrosine composition for a period until the underweight animal reaches the desired weight. The period is preferably at least 4 weeks, more preferably at least 6 weeks, and most preferably at least 8 weeks. Again, this period depends on the animal's original weight and the desired optimal weight trying to be achieved. In addition, the high tyrosine composition can be fed after the overweight animal has lost a substantial amount of weight as illustrated in Table 1.

Once the animal has achieved the desired weight, the animal can be fed the maintenance composition. The maintenance composition can be used for the remainder of the animal's life.

For the purposes of a complete understanding of the present invention it should be recognized that the term animal food composition is generally intended to apply to commercially sold and nutritionally complete and balanced animal food which provides the sole food intake for the animal.

The following examples and studies are intended to describe specific but nonlimiting embodiments of the present invention.

**STUDY 1:** Six adult cats (over 1 year of age) were fed ad libitum a canned cat food that was nutritionally complete and balanced for maintenance according to AAFCO's recommendation. The food was made by the following procedure:
1. Mix all the dry ingredients well.
2. Add the meat ingredients to the mixer while mixing.
3. Add the mixed dry ingredients to the mixer while mixing.
4. Add water to the mixer while mixing.
5. Mix the whole batch well, break any lumps formed before heating.
6. Cook the mixture to 180° F.
7. Fill cans with cooked mixture
8. Seam and cook the cans in a retort using typical retorting conditions for a canned cat food.

| **Ingredient** | % of ration |
|---|---|
| Dry Grain Mix | 25.438 |
| Meat Mix | 18.238 |
| Glycine | 0.700 |
| Vitamin Premix | 0.250 |
| Mineral Premix | 0.050 |
| Taurine | 0.100 |
| Water | 55.224 |
| TOTALS | 100.000 |

This canned food contained 0.08% tyrosine on an as fed basis (AS) or 0.3% tyrosine as dry matter basis (DM). The cats were fed this ration before they were given the same diet supplemented with tyrosine to 0.5% (AS, 1.93% DM) at the expense of glycine for 3 weeks. Afterwards, cats were provided with a commercial dry maintenance cat food (tyrosine: 0.93% AS) for 6 weeks.

**Results**: When on this diet, cats lost on average 0.95 kg body weight during a 10 week period, from 4.9 kg to 3.95 kg (Table 1). The average body weight was maintained at about 3.9 kg for three weeks when cats were given the same diet supplemented with tyrosine to 0.5% as fed basis (Table 1) even though cats were fed ad libitum. The average body weight increased continuously for six weeks after cats were fed the commercial dry cat food, clearly suggesting that more metobilized energy (ME) than needed to maintain the body weight was provided. When the cats were fed the same ration supplemented with tyrosine to 0.5% as fed basis (or 1.93% as dry matter basis) at the expense of glycine, cats stopped losing body weight and the body weight was maintained during the period of three-week feeding of the supplemented ration. Tyrosine supplemented ration significantly increased food and energy intake when compared with the canned ration without tyrosine supplement (Table 1). No other abnormality was observed during 19 weeks of the study period according to clinical observation and blood chemistry measurements. These results demonstrate that dietary tyrosine concentration can regulate body weight of cats through regulating appetite or food or energy intake.

Corresponding to dietary tyrosine and phenylalanine concentrations and food intake, the intake of both amino acids was low when low tyrosine ration was fed (Table 1).

Table 1 : Body weight, Food intake and nutrient intake

| Week | Diet | Body Weight | Food | DM | ME | Tyrosine | | Phenylalanine | | Tyrosine+ Phenylalanine | |
|---|---|---|---|---|---|---|---|---|---|---|---|
| | | kg | g/d | g/d | Kcal/d | g/d | g/kg BW | g/d | g/kg BW | g/d | g/kg BW |
| 1 | Low Tyr | 4.9 | 145.9 | 36.7 | 139.2 | 0.12 | 0.02 | 0.32 | 0.07 | 0.44 | 0.09 |
| 2 | Low Tyr | 4.8 | 137.1 | 34.5 | 130.7 | 0.11 | 0.02 | 0.30 | 0.06 | 0.41 | 0.09 |
| 3 | Low Tyr | 4.6 | 131.1 | 33.0 | 125.0 | 0.10 | 0.02 | 0.29 | 0.06 | 0.39 | 0.09 |
| 4 | Low Tyr | 4.5 | 143.5 | 36.1 | 136.9 | 0.11 | 0.03 | 0.32 | 0.07 | 0.43 | 0.10 |
| 5 | Low Tyr | 4.5 | 156.2 | 39.3 | 149.0 | 0.12 | 0.03 | 0.34 | 0.08 | 0.47 | 0.11 |
| 6 | Low Tyr | 4.4 | 135.9 | 34.2 | 129.6 | 0.11 | 0.03 | 0.30 | 0.07 | 0.41 | 0.09 |
| 7 | Low Tyr | 4.3 | 125.5 | 31.6 | 119.7 | 0.10 | 0.02 | 0.28 | 0.07 | 0.38 | 0.09 |
| 8 | Low Tyr | 4.2 | 137.8 | 34.7 | 131.5 | 0.11 | 0.03 | 0.30 | 0.07 | 0.41 | 0.10 |
| 9 | Low Tyr | 4.1 | 157.3 | 39.6 | 150.0 | 0.13 | 0.03 | 0.35 | 0.08 | 0.47 | 0.12 |
| 10 | Low Tyr | 4.1 | 143.8 | 36.2 | 137.2 | 0.12 | 0.03 | 0.32 | 0.08 | 0.43 | 0.11 |
| 11 | High Tyr | 4.0 | 169.5 | 43.8 | 164.9 | 0.86 | 0.22 | 0.39 | 0.10 | 1.25 | 0.32 |
| 12 | High Tyr | 3.9 | 167.0 | 43.2 | 162.4 | 0.85 | 0.22 | 0.38 | 0.10 | 1.24 | 0.31 |
| 13 | High Tyr | 3.9 | 176.2 | 45.6 | 171.4 | 0.90 | 0.23 | 0.41 | 0.10 | 1.30 | 0.33 |
| 14 | Dry Mail1t. | 4.1 | 71.3 | 65.2 | 291.6 | 0.66 | 0.17 | 1.02 | 0.26 | 1.68 | 0.43 |
| 15 | Dry Maint. | 4.2 | 63.7 | 58.4 | 260.8 | 0.59 | 0.14 | 0.91 | 0.22 | 1.50 | 0.36 |
| 16 | Dry Maint. | 4.3 | 63.5 | 58.1 | 259.8 | 0.59 | 0.14 | 0.91 | 0.22 | 1.50 | 0.36 |
| 17 | Dry Maint. | 4.3 | 68.6 | 62.8 | 280.7 | 0.64 | 0.15 | 0.98 | 0.23 | 1.62 | 0.38 |
| 18 | Dry Maint. | 4.4 | 72.4 | 66.2 | 296.1 | 0.67 | 0.16 | 1.03 | 0.24 | 1.71 | 0.40 |
| 19 | Dry Maint. | 4.5 | 71.3 | 65.3 | 291.7 | 0.66 | 0.15 | 1.02 | 0.23 | 1.65 | 0.38 |
| Paired t-test, p values | | | | | | | | | | | |
| Wk 1 | | | | | | | | | | | |
| vs wk | | | | | | | | | | | |
| 10 | | <0.01 | 0.935 | 0.935 | 0.935 | 0.935 | 0.377 | 0.935 | 0.377 | 0.935 | 0.377 |
| Wk | | | | | | | | | | | |
| 10 vs | | | | | | | | | | | |
| wk 11 | | 0.480 | 0.014 | 0.007 | 0.009 | <0.01 | <0.01 | 0.005 | 0.004 | <0.01 | <0.01 |
| Wk | | | | | | | | | | | |
| 10 vs | | | | | | | | | | | |
| wk 13 | | 0.621 | 0.015 | 0.009 | 0.011 | <0.01 | <0.01 | 0.007 | 0.006 | <0.01 | <0.01 |
| Wk | | | | | | | | | | | |
| 11 vs | | | | | | | | | | | |
| wk 13 | | 0.054 | 0.325 | 0.325 | 0.325 | 0.325 | 0.221 | 0.325 | 0.221 | 0.325 | 0.221 |
| Wk | | | | | | | | | | | |
| 10 vs | | | | | | | | | | | |
| wk 14 | | | | <0.01 | <0.01 | <0.01 | <0.01 | <0.01 | <0.01 | <0.01 | <0.01 |
| Wk | | | | | | | | | | | |
| 13 vs | | | | | | | | | | | |
| wk 14 | | | | 0.003 | <0.01 | 0.023 | 0.008 | <0.01 | <0.01 | 0.016 | 0.021 |
| Wk | | | | | | | | | | | |
| 14 vs | | | | | | | | | | | |
| wk 19 | | | | 0.992 | 0.992 | 0.992 | 0.012 | 0.992 | 0.012 | 0.992 | 0.012 |

Low dietary tyrosine was also reflected in the serum tyrosine concentration (Table 2).

| Table 2. Serum tyrosine and phenylalanine concentration (µmol/ml) in cats | | |
|---|---|---|
| Weeks | Tyrosine | Phenylalanine |
| 0 | 0.050 | 0.069 |
| 5 | 0.024 | 0.060 |
| 13 | 0.056 | 0.057 |

| Pared t-test p values | | |
|---|---|---|
| wk 0 vs. 5 | 0.001 | 0.016 |
| wk 0 vs. 13 | 0.501 | 0.149 |
| wk 5 vs. 13 | 0.005 | 0.678 |

Serum tyrosine concentration was significantly lower after cats were fed the low tyrosine ration for 5 weeks. There was no significant difference in serum phenylalanine concentration between week 5 and week 13..

The body weight loss in cats fed the low tyrosine ration was due to insufficient ME intake although they were fed ad libitum. Weekly average ME intake was about 130 kcal/day (Table 1), which is below the requirement calculated from the formula: Daily Energy Requirement (kcal/day) = 70 x Body Weight (kg) ^{0.75} x 1.2. Based on the formulation, the daily energy requirement for a neutered adult cat of 4.9 kg is 277 kcal/day. When cats were given the tyrosine-supplemented ration, weekly average ME intake increased to about 160 kcal/day (Table 1), which was still below the calculated daily energy requirement (233 kcal/day for body weight of 3.9 kg), but the body weight was stabilized, suggesting ME requirement was met (Table 1). Weekly average ME intake was 280 kcal/day when cats were fed the commercial dry cat food (Table 1), higher than the calculated daily energy requirement (233 kcal/day for body weight of 3.9 kg). Therefore, it is not surprising to see the increase in the body weight.

Tyrosine appears to be involved in the energy intake regulation. When dietary tyrosine was 0.08% (0.3% DM), it was not sufficient for cats to ingest enough energy to maintain the body weight. When dietary tyrosine increased to 0.5% (1.93% DM), cats were able to consume enough energy to maintain the body weight.

Tyrosine is a dispensable amino acid and can be produced in mammals by phenylalanine hydroxylation. Tyrosine is a precursor of neurotransmitters such as catecholamines and a precursor of pigments such as eumelanins and pheomelanin. Controlling dietary tyrosine and/or phenylalanine may alter catecholamine level in central nervous system (CNS) and may control food or energy intake. Therefore, controlling dietary tyrosine and/or phenylalanine concentration can be used in food intake regulation and body weight control in overweight and obese subjects.

No clinical abnormalities were observed in cats fed the low tyrosine ration except the body weight loss.

Complete blood count (CBC) and serum chemistry parameters were within the normal range except mean corpuscular hemoglobin concentration (MCHC) (33.7%) and alanine aminotransferase (ALT) (94.2 u/1) which exceeded the maximum of the normal ranges after cats were fed the low tyrosine ration for 10 weeks. There was no difference (p> 0.05) in ALT measured at week 0 (before feeding the low tyrosine ration), week 10 (after feeding the low tyrosine ration for 10 weeks), and week 13 (after feeding the tyrosine-supplemented ration for 3 weeks).

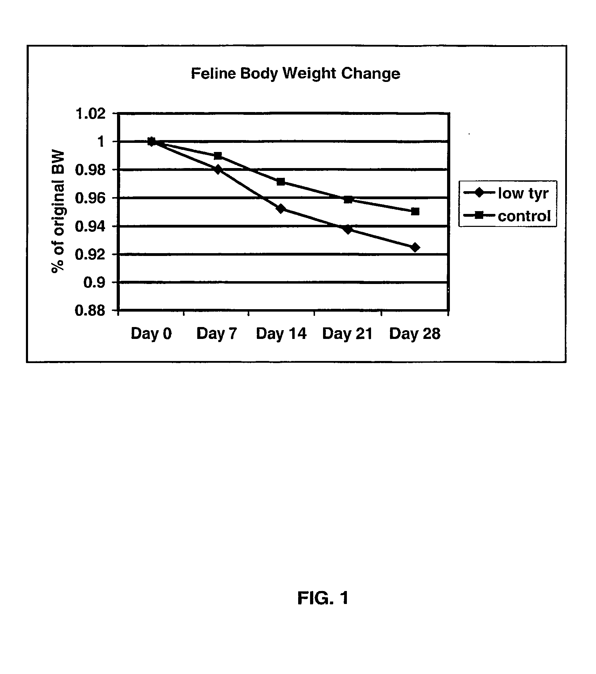

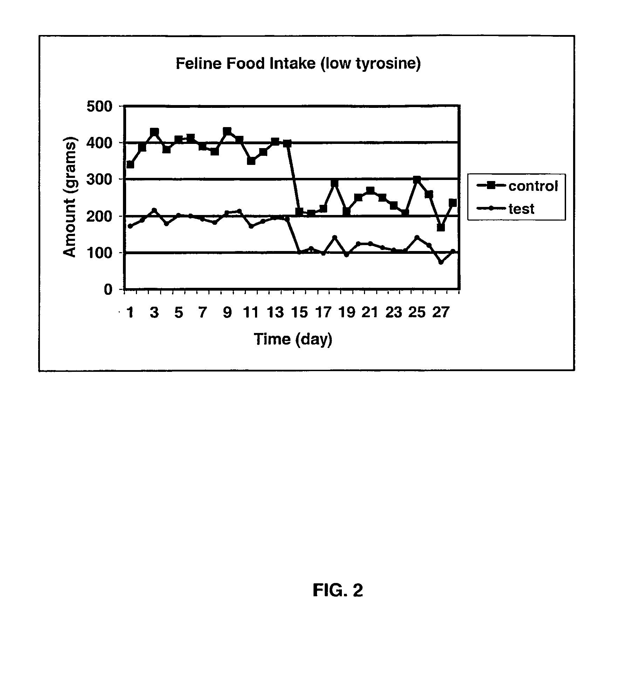

**STUDY 2:** Twenty adult cats (over 1 year of age) were fed ad libitum one of two canned cat foods that was nutritionally complete and balanced for maintenance according to AAFCO's recommendation for 4 weeks. This canned food contained 0.35% tyrosine and 0.82% phenylalanine on a dry matter basis. When on this ration, cats lost on average 7.2% of their initial body weight or 0.42 kg (Figure 1). Cats fed the tyrosine supplemented food (2.7% tyrosine and 0.96% phenylalanine) only lost an average of 1% of their initial body weight (e.g. 0.06 kg). Cats fed the low tyrosine food had significantly lower food intakes (average 151.5 g/day) than the cats fed the tyrosine supplemented control food (162.2 g/day) (Figure 2). No other abnormality was observed during the 4 week time period. These results further demonstrate that dietary tyrosine concentration can regulate body weight of cats through regulating appetite or food intake.

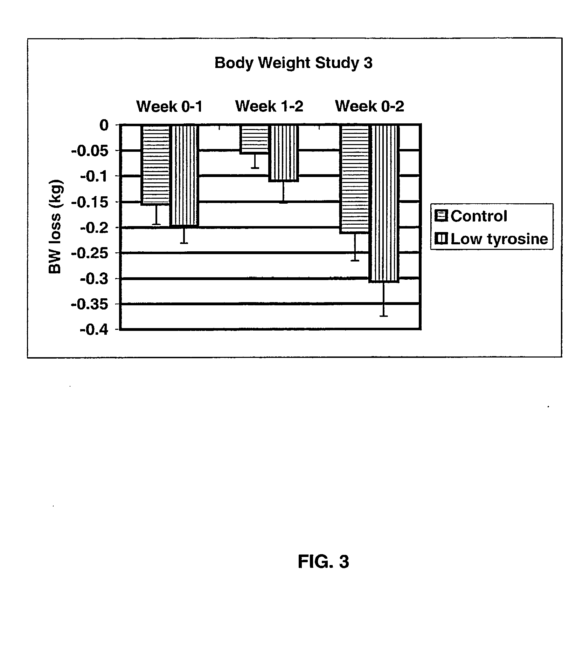

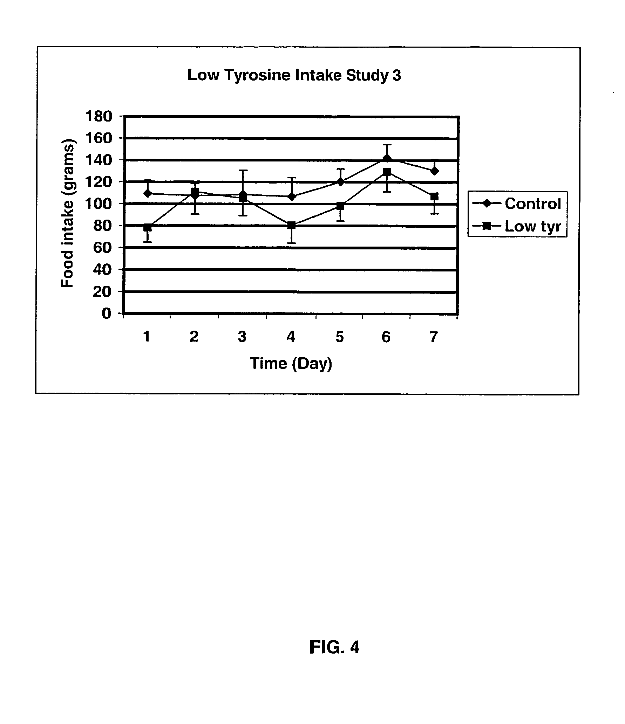

**STUDY 3:** Twenty adult cats (over 1 year of age) were fed ad libitum one of two canned cat foods that was nutritionally complete and balanced for maintenance according to AAFCO's recommendation for 2 weeks. These foods were formulated to have higher protein levels than the food tested in study 2 (protein level on a dry matter basis: study 2 =33.21 % & study 3 =34.91%. This canned food contained 0.34% tyrosine and 0.89% phenylalanine on a dry matter basis. When on this ration, cats lost on average 6.7% of their initial body weight or 0.31 kg (Figure 3). Cats fed the tyrosine supplemented (1.95% tyrosine and 0.80% phenylalanine on a dry matter basis) only lost an average of 4.3% of their initial body weight (e.g. 0.21 kg). Cats fed the low tyrosine food had lower food intakes than the cats fed the tyrosine supplemented control food (Figure 4). These results further demonstrate the concept that dietary tyrosine concentration can regulate body weight of cats through regulating appetite or food intake.

STUDY 4: A dry feline food was formulated that contained 26.8% protein, 0.36% tyrosine, and 0.64% phenylalanine on a dry matter basis. This food was fed to 10 cats ad libitum for 4 consecutive weeks while 10 different cats were fed a commercial light cat food (control food) for the same time period. Body weights were recorded weekly and food consumption was recorded daily. Cats fed the low tyrosine food lost weight each week whereas those fed the control food gained weight (Table 3). Likewise, cats fed the low tyrosine food consumed significantly less food than the cats fed the control food (Table 4). These results confirm those of previous studies and also show that the concept is efficacious in a dry formulation.

**TABLE 3 Body weight change (%, Mean± SEM)**

| | Week 0-1 | Week 1-2 | Week 2-3 | Week 3-4 |
|---|---|---|---|---|
| Low tyrosine food | -2.10±1.10 | -1.75±1.44 | -3.10±1.99 | -1.61±0.66 |
| Control food | 0.67±1.26 | 1.73±1.05 | 2.33±1.22 | 2.28±1.06 |

**TABLE 3 Weekly food consumption (g, Mean±SEM)**

| | Week 0-1 | Week 1-2 | Week 2-3 | Week 3-4 | Total |
|---|---|---|---|---|---|
| Low tyrosine food | 75.81±4.58 | 69.59±3.90 | 71.22±3.05 | 65.22±2.78 | 70.46±5.57 |
| Control food | 81.24±7.9 | 83.89±4.61 | 84.74±5.00 | 73.19±3.70 | 80.76±4.38 |

**RANGE OF EFFECTIVE TYROSINE LEVELS:**

| STUDIES | % tyrosine for low tyrosine composition (dry matter basis) |
|---|---|
| 1 | 0.30 |
| 2 | 0.35 |
| 3 | 0.34 |
| 4 | 0.36 |

## Claims

1. An edible composition which comprises tyrosine in an amount not to exceed about 0.4% by weight on a dry matter basis, for use in reducing the body weight of an animal.

2. The composition as claimed in claim 1, wherein said tyrosine is in an amount from about 0.08 to about 0.38% by weight on a dry matter basis.

3. The composition as claimed in claim 1, wherein said tyrosine is in an amount from about 0.2 to about 0.36% by weight on a dry matter basis.

4. An edible composition, which comprises on a dry matter basis
(a) from 0.01 % to less than 0.4% by weight of tyrosine;
(b) from 0.1% up to just below the toxic level of phenylalanine, and preferably in an amount from 0.42 to 3% by weight,
(c) from 7% to 70% by weight, and preferably from 30 to 50% by weight of protein;
(d) from about 1% to 60% by weight and preferably from 20 to 50% by weight of fat;
(e) from 0 to 90% by weight, and preferably from 10 to 50% by weight on a dry matter basis of carbohydrate;
(f) from 0 to 40% by weight, and preferably from 5 to 25% by weight on a dry matter basis, of dietary fiber; and
(g) from 0 to about 15% by weight, and preferably from about 0.1 to about 4% by weight on a dry matter basis, of nutritional balancing agents.

5. The composition as claimed in any one of the preceding claims, wherein said tyrosine is from gelatin, lungs, whey or grains.

6. The composition as claimed in any one of the preceding claims, wherein said composition is a treat, an animal food or supplement.

7. The composition as claimed in claim 6, wherein said composition is a canine food, or a feline food, or a farm animal food, or a hog food.

8. Use of tyrosine for the production of an animal food for controlling the body weight of an animal, which comprises determining if the animal is an overweight or underweight animal and feeding the overweight animal an animal food which comprises the composition as claimed in claim 4, with a low tyrosine content, for a period until said animal reaches the desired weight; or feeding the underweight animal an animal food with a high tyrosine content based on a dry matter basis of at least 1.2% by weight for a period until said animal reaches the desired weight.

9. Use as claimed in claim 8, wherein said animal is fed the animal food for a period of at least 4 weeks.

10. Use as claimed in claim 8 or 9, which further requires feeding said animal a maintenance ration after the animal has achieved the desired weight wherein the maintenance ration has an amount of tyrosine between the level of the low tyrosine content for the overweight animal and the high tyrosine content for the underweight animal.

11. Use as claimed in claim 10, wherein the maintenance ration is fed once a day for at least 4 weeks.

12. Use as claimed in any one of claims 8-11, wherein said animal is a feline, a canine, a farm animal, or a hog.

13. Use of tyrosine for the production of an animal food which contains a composition as claimed in claim 4 or claim 5 for reducing food intake and body weight of an overweight and obese animal, wherein the animal is fed for a period of time such that said animal loses weight.

14. Use as claimed in claim 13, wherein the period is for at least 4 weeks, or for at least 8 weeks.

15. Use as claimed in claim 13 or 14, which further requires feeding said animal a maintenance ration after the animal has achieved the desired weight wherein the maintenance ration has an amount of tyrosine greater than the level of the low tyrosine content for said overweight animal.

16. Use of tyrosine for the production of an animal food for increasing food intake and body weight of an underweight animal, which animal food comprises tyrosine in an amount of from 1.2% by weight to just below the toxic level of tyrosine or a dry matter basis in the food.

## Patentansprüche

1. Essbare Zusammensetzung, die Tyrosin in einer Menge umfasst, die etwa 0,4 Gew.-%, auf Basis der Trockenmasse, nicht überschreitet, zur Verwendung bei der Verminderung des Körpergewichts eines Tieres.

2. Zusammensetzung nach Anspruch 1, wobei das Tyrosin in einer Menge von etwa 0,08 bis etwa 0,38 Gew.-%, auf Basis der Trockenmasse, ist.

3. Zusammensetzung nach Anspruch 1, wobei das Tyrosin in einer Menge von etwa 0,2 bis etwa 0,36 Gew.-%, auf Basis der Trockenmasse, ist.

4. Essbare Zusammensetzung, die, auf Basis der Trockenmasse, umfasst:
(a) 0,01 bis weniger als 0,4 Gew.-% Tyrosin,
(b) 0,1 bis genau unterhalb des toxischen Schwellwerts an Phenylanalin, und vorzugsweise in einer Menge von 0,42 bis 3 Gew.-%,
(c) 7 Gew.-% bis 70 Gew.-% und vorzugsweise 30 bis 50 Gew.-% Protein,
(d) etwa 1 Gew.-% bis 60 Gew.-% und vorzugsweise 20 bis 50 Gew.-% Fett,
(e) 0 bis 90 Gew.-%, und vorzugsweise 10 bis 50 Gew.%, auf Basis der Trockenmasse, an Kohlenhydrat,
(f) 0 bis 40 Gew.-%, und vorzugsweise 5 bis 25 Gew.-%, auf Basis von Trockenmasse, an Ballaststoff und
(g) 0 bis etwa 15 Gew.-%, und vorzugsweise etwa 0,1 bis etwa 4 Gew.-%, auf Basis von Trockenmasse, an Mittel für die Ausgewogenheit der Ernährung.

5. Zusammensetzung nach einem der vorhergehenden Ansprüche, wobei das Tyrosin aus Gelatine, Lunge, Molke oder Korn ist.

6. Zusammensetzung nach einem der vorhergehenden Ansprüche, wobei die Zusammensetzung eine Leckerei, ein Tierfutter oder Tierergänzungsmittel ist.

7. Zusammensetzung nach Anspruch 6, wobei die Zusammensetzung ein Hundefutter, oder ein Katzenfutter, oder ein Nutztierfutter, oder ein Schweinefutter ist.

8. Verwendung von Tyrosin für die Herstellung eines Tierfutters zur Kontrolle des Körpergewichts eines Tieres, wobei bestimmt wird, ob das Tier ein übergewichtiges oder untergewichtiges Tier ist und dem übergewichtigen Tier ein Tierfutter, das die Zusammensetzung gemäß Anspruch 4 mit einem niedrigen Tyrosingehalt umfasst, für einen Zeitraum, bis das Tier das gewünschte Gewicht erreicht, gegeben wird oder dem untergewichtigen Tier ein Tierfutter mit einem hohen Tyrosingehalt auf der Basis von mindestens 1,2 Gew.-% auf Basis von Trockenmasse für einen Zeitraum, bis das Tier das gewünschte Gewicht erreicht, gegeben wird.

9. Verwendung nach Anspruch 8, wobei das Tierfutter dem Tier für einen Zeitraum von mindestens 4 Wochen gegeben wird.

10. Verwendung nach Anspruch 8 oder 9, bei dem dem Tier eine Erhaltungsration gegeben wird, nachdem das Tier das gewünschte Gewicht erreicht hat, wobei die Erhaltungsration eine Menge von Tyrosin zwischen dem Niveau des niedrigen Tyrosingehalts für das übergewichtige Tier und dem hohen Tyrosingehalt für das untergewichtige Tier hat.

11. Verwendung nach Anspruch 10, wobei die Erhaltungsration einmal täglich mindestens 4 Wochen lang gegeben wird.

12. Verwendung nach einem der Ansprüche 8 bis 11, wobei das Tier eine Katze, ein Hund, ein Nutztier oder ein Schwein ist.

13. Verwendung von Tyrosin zur Herstellung eines Tierfutters, das eine Zusammensetzung gemäß Anspruch 4 oder Anspruch 5 enthält, zur Verringerung der Futteraufnahme und des Körpergewichts eines übergewichtigen und fettleibigen Tiers, wobei das Tier für einen Zeitraum gefüttert wird, so dass das Tier Gewicht verliert.

14. Verwendung nach Anspruch 13, wobei der Zeitraum mindestens 4 Wochen oder mindestens 8 Wochen beträgt.

15. Verwendung nach Anspruch 13 oder 14, bei der dem Tier eine Erhaltungsration gegeben wird, nachdem das Tier das gewünschte Gewicht erreicht hat, wobei die Erhaltungsration eine Menge an Tyrosin hat, die größer ist als die Menge des niedrigen Tyrosingehalts für das übergewichtige Tier.

16. Verwendung von Tyrosin zur Herstellung eines Tierfutters zur Erhöhung der Futteraufnahme und des Körpergewichts eines untergewichtigen Tiers, wobei das Tierfutter Tyrosin in einer Menge von 1,2 Gew.-% bis genau unterhalb des toxischen Schwellwerts an Tyrosin, auf Basis von Trockenmasse in dem Futter, umfasst.

## Revendications

1. Composition comestible qui comprend de la tyrosine en une quantité ne dépassant pas environ 0,4 % en poids par rapport à la matière sèche, pour une utilisation dans la réduction du poids corporel d'un animal.

2. Composition selon la revendication 1, dans laquelle ladite tyrosine se trouve en une quantité d'environ 0,08 à environ 0,38 % en poids par rapport à la matière sèche.

3. Composition selon la revendication 1, dans laquelle ladite tyrosine se trouve en une quantité d'environ 0,2 à environ 0,36 % en poids par rapport à la matière sèche.

4. Composition comestible, qui comprend par rapport à la matière sèche
(a) de 0,01 à moins de 0,4 % en poids de tyrosine ;
(b) de 0,1 % jusqu'à juste au-dessous du niveau toxique de phénylalanine, et de préférence en une quantité de 0,42 à 3 % en poids ;
(c) de 7 à 70 % en poids, et de préférence de 30 à 50 % en poids de protéines ;
(d) d'environ 1 à 60 % en poids, et de préférence de 20 à 50 % en poids de graisses ;
(e) de 0 à 90 % en poids, et de préférence de 10 à 50 % en poids par rapport à la matière sèche de glucides ;
(f) de 0 à 40 % en poids, et de préférence de 5 à 25 % en poids par rapport à la matière sèche, de fibres diététiques ; et
(g) de 0 à environ 15 % en poids, et de préférence d'environ 0,1 à environ 4 % en poids par rapport à la matière sèche, d'agents d'équilibrage nutritif.

5. Composition selon l'une quelconque des revendications précédentes, dans laquelle ladite tyrosine provient de la gélatine, des poumons, du petit lait ou de graines.

6. Composition selon l'une quelconque des revendications précédentes, dans laquelle ladite composition est une friandise, un aliment ou un complément animal.

7. Composition selon la revendication 6, dans laquelle ladite composition est un aliment canin, ou un aliment félin, ou un aliment d'animal de ferme ou un aliment porcin.

8. Utilisation de la tyrosine pour la production d'un aliment animal pour contrôler le poids corporel d'un animal, qui comprend de déterminer si l'animal est un animal en surpoids ou en sous-poids et d'alimenter l'animal en surpoids avec un aliment animal qui comprend la composition selon la revendication 4, avec une faible teneur en tyrosine, durant une période jusqu'à ce que l'animal atteigne le poids souhaité ; ou d'alimenter un animal en sous-poids avec un aliment animal avec une teneur en tyrosine élevée par rapport à la matière sèche d'au moins 1,2 % en poids durant une période jusqu'à ce que l'animal atteigne le poids souhaité.

9. Utilisation selon la revendication 8, dans laquelle ledit animal est alimenté avec l'aliment animal durant une période d'au moins 4 semaines.

10. Utilisation selon la revendication 8 ou 9, qui nécessite en outre l'alimentation dudit animal avec une ration d'entretien après que l'animal ait atteint le poids souhaité où la ration d'entretien a une quantité de tyrosine entre le niveau de la faible teneur en tyrosine pour l'animal en surpoids et la teneur en tyrosine élevée pour l'animal en sous-poids.

11. Utilisation selon la revendication 10, dans laquelle la ration d'entretien est fournie une fois par jour durant au moins 4 semaines.

12. Utilisation selon l'une quelconque des revendications 8 à 11, dans laquelle ledit animal est un animal félin, canin, un animal de ferme ou porcin.

13. Utilisation de la tyrosine pour la production d'un aliment animal qui contient une composition selon la revendication 4 ou la revendication 5 pour réduire l'ingestion alimentaire et le poids corporel d'un animal en surpoids et obèse, dans lequel on alimente l'animal durant une période de temps telle que ledit animal perd du poids.

14. Utilisation selon la revendication 13, dans laquelle la période dure au moins 4 semaines, ou au moins 8 semaines.

15. Utilisation selon la revendication 13 ou 14, qui nécessite en outre l'alimentation dudit animal avec une ration d'entretien après que l'animal ait atteint le poids souhaité, dans laquelle la ration d'entretien a une quantité de tyrosine supérieure au niveau de faible teneur en tyrosine pour ledit animal en surpoids.

16. Utilisation de la tyrosine pour la production d'un aliment animal pour augmenter l'ingestion d'aliments et le poids corporel d'un animal en sous-poids, lequel aliment animal comprend de la tyrosine en une quantité de 1,2 % en poids jusque juste au-dessous du niveau toxique de tyrosine ou par rapport à la matière sèche dans l'aliment.
